# EUROPEAN PATENT SPECIFICATION

(11) **EP 3 860 966 B1**
(45) Date of publication and mention of the grant of the patent: **18.06.2025**
(21) Application number: 19797799.4
(22) Date of filing: 04.10.2019
(51) Int. Cl.: C07C 7/00, C07C 7/04, C07C 7/10, C07C 7/12, C07C 7/148, C07C 67/03, C07C 5/03

(54) **SQUALENE OF HIGH QUALITY PRODUCED BY MICROWAVE ASSISTED PROCESS**
DURCH MIKROWELLENUNTERSTÜTZTES VERFAHREN HERGESTELLTES QUALITATIV HOCHWERTIGES SQUALEN
SQUALÈNE DE HAUTE QUALITÉ PRODUIT PAR UN PROCÉDÉ ASSISTÉ PAR MICO-ONDES

(30) Priority: 05.10.2018 DE 102018124593
(43) Date of publication of application: 11.08.2021
(73) Proprietor: Scholz, Gertraud, 63755 Alzenau (DE)
(72) Inventor: SCHOLZ, Gertraud, 63755 Alzenau (DE); CRAVOTTO, Giancarlo, 10137 Torino (IT); SCHOLZ, Hans Jürgen, 63755 Alzenau (DE)
(74) Representative: Völger, Karl Wolfgang
(86) International application number: PCT/IB2019/058477
(87) International publication number: WO 2020/070707

(56) References cited:
- WO-A1-2015/197155
- US-A- 6 165 481

## Description

The present disclosure relates to squalene of high quality produced by a microwave assisted continuous process of plant-based material and the use of squalene and squalane in cosmetics, nutrition and health care. The process can be performed in volumes of industrial relevance.

Squalene ((E)-2,6,10,15,19,23-Hexamethyl-2,6,10,14,18,22-tetracosahexaene; IUPAC), a highly unsaturated hydrocarbon (C₃₀H₅₀) according Formula I is found throughout nature such as in vegetable oils, particularly in olive oil, soya oil, sunflower oil, rapeseed oil, rice bran oil or wheat germ oil, and can be found in high concentrations in fish oil, particularly in shark liver oil (40 % - 90 %).

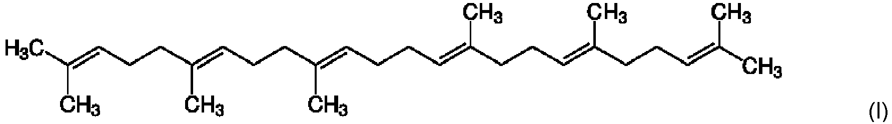

Squalene is known as an intermediate in the biosynthesis of phytosterol or cholesterol and is used in the pharmaceutical industries as well as a natural dietary supplement in nutrition.

Shark liver oil remains the richest natural source of squalene. Because of the presence of different persistent organic pollutants (POPs) in the sea environment, like PCB (polychlorinated biphenyl), PBDE (polybrominated diphenyl ether), organochlorine pesticides, polycyclic aromatic hydrocarbons, dioxin, and heavy metals, together with the concern in preservation of marine life, there is today a great interest in finding new natural sources for squalene, especially vegetal ones.

In general, squalene has been obtained from plant-based oils comprising squalene by using organic solvents such as hexane or supercritical fluids for extraction processing, by distillation or by chromatography.

WO 2015/197155 A1 describes a continuous process for concentration and isolation of tocopherols, sterols and/or terpenes from oily or fatty mixtures of biological origin, particularly the isolation of tocopherol in a microwave assisted process. For example, olive oil DDO (Deodorisation Distillates Oils), comprising fatty acid as the main component, besides glyceride and the unsaponifiable minor components such as tocopherol and squalene was reacted with methanol catalysed by sulfuric acid. According to the reply to communication from the EPO Examination Division (dated May 4, 2018) regarding the patent application EP 3 160 953 (EP counterpart of WO 2015/197155 A1) on page 5, 70 % of isomerisation of squalene was detected by GC showing unfavourable reaction conditions for isolating squalene of high quality.

To avoid the use of toxic solvents like hexane and the exposure of the plant-based oil to high temperatures for long periods of time causing partly decomposition or isomerisation of squalene, there is still a need for a new economic process to produce squalene and/ or squalane with high quality from plant materials for the use in nutrition, pharmaceuticals and cosmetics.

It is an object of the present invention to provide a process for obtaining a product comprising squalene based on oily or fatty mixtures of plant origin, characterised by high purity and high concentrations.

A product comprising squalene according to Formula I in an amount of equal or more than 50 wt.-% related to the total weight of the product, 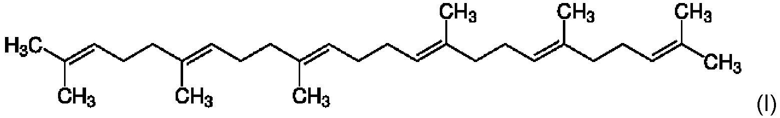 can be produced by a continuous flow, microwave assisted process specified in detail below.

Surprisingly it has been found that in contrast to the prior art teaching aqueous methanol or aqueous ethanol as reactant has a positive influence on the reaction, and isomerisation of squalene is significantly suppressed. To be specific, according to the present invention isomerisation of squalene is significantly reduced from 70 % isomerisation to 50 % and up to 0 % isomerization compared to the state of the art, resulting in a product containing squalene according to Formula I in concentrations of 50.0 wt.-% to 99.9 wt.-%, related to the total weight of the product, produced by the inventive process.

Furthermore, the aqueous methanol or aqueous ethanol shows a higher absorption rate of microwave energy compared to pure methanol or ethanol. This results in a very fast heating rate of the reaction mixture. The methanol or ethanol can be reused in the continuous process without removal of water in a drying step.

The product obtained by the process according to the present invention comprises squalene in higher concentration compared to the prior art. Further, it contains low levels of by-products of squalene such as isomerisation products of squalene in the range of 30 wt.-% to 0.0 wt.-%, preferably in the range of 30 wt.-% to 0.5 wt.-%, more preferred 20 wt.-% to 0.1 wt.-%, particularly preferred in the range of 10 wt.-% to 0.0 wt.-%, related to the total weight of the inventive product. Therein, the boundary value of 0.0 wt.-% is confined by the detection limit which technically feasible.

It is particularly preferred if the reaction mixture is free of sulphur containing acids. Acids like sulfuric acid, p-toluene sulfonic acid, methane sulfonic acid enhance isomerization. Traces of sulphur in the fatty acid ester fraction can negatively influence subsequent processes like hydrogenation fatty acid methyl or fatty acid ethyl esters to fatty alcohols.

The product comprising squalene in concentrations of 50.0 wt.-% to 99.9 wt.-%, preferably 60.0 wt.-% to 99.8 wt.-%, related to the total weight of the product, produced by the inventive process is a nearly colourless liquid with low viscosity.

In a further development the oily or fatty material of plant origin is selected from olive oil, soybean oil, sunflower oil, palm oil, rapeseed oil, hazelnuts oil, peanuts oil, corn oil and Deodoriser Distillate (DD)-vegetable oils.

In a preferred embodiment the aqueous methanol or the aqueous ethanol used in step a) comprises water in the range of 1 wt.-% to 99 wt.-%, preferably 2 wt.-% to 80 wt.-%, more preferably 3 wt.-% to 50 wt.-% and particularly preferred 5 wt.-% to 30 wt.-%, related to the total amount of the used aqueous methanol or aqueous ethanol. This leads to a significant reduction of isomerization or decomposition of squalene. Energy savings are realized by avoidance of an alcohol drying step.

In another preferred embodiment, the product comprises squalene according to Formula I in an amount of 50.0 wt.-% to 99.9 wt.-%, preferably 60.0 wt.-% to 99.8 wt.-% related to the total weight of the product. The obtained squalene in an amount of 50.0 wt.-% to 99.9 wt.-% can be separated or isolated in a following work-up process step very efficiently as there is no negative impact of impurities.

It is furthermore preferred if the wt.-% ratio of oily or fatty material to alcohol is 15 to 1 to 1 to 3, preferably 10 to 1 to 1 to 2, more preferably 5 to 1 to 1 to 1, in step a).

Advantageously the reaction speed is accelerated, resulting in short reaction times, high conversion rates to fatty acid methylesters or fatty acid ethylesters and a low free acid level (acid number < 1 mg KOH/g) of the reaction product mixture.

The above statements and preferences for both the products of the invention are valid for the process of the invention, described below, as well. Similarly, the observations and preferences below for the process of the invention are valid correspondingly for both the products of the invention.

The object of the present invention, identified above, has been achieved in the present invention by a continuous flow, microwave assisted process for producing a product comprising squalene according to Formula I in an amount of equal or more than 50 wt.-% related to the total weight of the product, 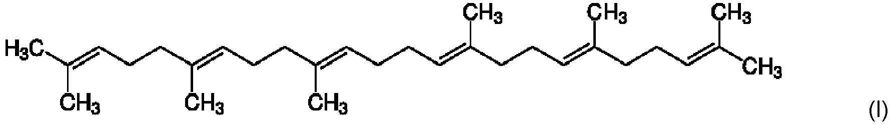 the process comprising the steps
a) preparing a reaction mixture containing an oily or fatty material of plant origin and aqueous methanol or aqueous ethanol,
b) continuously conveying of the reaction mixture through a microwave-transparent reaction zone and irradiating the reaction mixture with microwaves inside the reaction zone in the presence of an acidic catalyst, whereby the residence time of the reaction mixture in the microwave-transparent reaction zone is 1 s to 180 s, the temperature in the reaction zone being equal to or between 100 °C and 220 °C, the pressure in the reaction zone being 1.5 bar to 30 bar,
   wherein a reaction of the reaction mixture takes place in the reaction zone resulting in a mixture of polar substances comprising glycerol, methanol or ethanol, and water, and non-polar substances comprising aqueous fatty acid methylesters or fatty acid ethylesters and unsaponifiable components,
c) releasing the pressure to ambient pressure,
d) after phase separation the polar phase is separated from the non-polar phase containing the non-polar substances,
e) separating aqueous fatty acid methylesters or fatty acid ethylesters from the non-polar phase by falling film evaporation or thin-layer evaporation,
f) separating squalene according to Formula I from the unsaponifiable components by molecular distillation, short-pass distillation, extraction or freeze separation.

The process of the present invention generally shares the advantages of the above-described product. In particular, it has been found that in contrast to the prior art teaching aqueous methanol or aqueous ethanol as reactant has a positive influence on the reaction, and isomerisation of squalene is significantly suppressed. To be specific, according to the present invention isomerisation of squalene is significantly reduced from 70 % isomerisation to 50 % and up to 0 % isomerization of squalene compared to the state of the art, resulting in a product containing squalene according to Formula I in concentrations of 50.0 wt.-% to 99.9 wt.-%, related to the total weight of the product. Again, the boundary value of 0.0 wt.-% is confined by the detection limit which technically feasible.

The aqueous methanol or aqueous ethanol shows a higher absorption rate of microwave energy compared to pure methanol or ethanol. This results in a very fast heating rate of the reaction mixture. The methanol or ethanol can be reused in the continuous process without removal of water in a drying step.

In the inventive process, using a homogeneous or a heterogeneous catalyst as well as a supported catalyst on a carrier is preferred.

Using a homogenous catalyst, the catalyst is mixed in step a) of the process with an oily or fatty material of plant origin and aqueous methanol or aqueous ethanol.

In a further development of the inventive process, the homogeneous catalyst is selected from phosphoric acid, phosphonic acid, nitric acid, organic carboxylic acid, lactic acid, citric acid, aluminium sulfate hydrate, Alum, acidic silica gel or acidic aluminum hydroxide, aluminates Al(OR)₃ and titanates Ti(OR)₄, in which the groups R can be identical or different, independently from each other and being selected of C₁-C₁₀-Alkyl groups, for example methyl, ethyl, n-propyl, iso-propyl, n-butyl, iso-butyl, sec.-butyl, tert-butyl, n-pentyl, iso-pentyl, sec-pentyl, neo-pentyl, 1,2-dimethylpropyl, iso-amyl, n-hexyl, sec-hexyl, n-heptyl, n-octyl, 2-ethylhexy, n-nonyl or n-decyl, from C3-C12-cycloalkyl groups, for example cyclopropyl, cyclobutyl, cyclopentyl, cyclohexyl, cycloheptyl, cyclooctyl, cyclononyl, cyclodecyl, cycloundecyl und cyclododecyl; preferred are cyclopentyl, cyclohexyl und cycloheptyl. The preferred the groups R in Al(OR)₃ or Ti(OR)₄ are identical and selected from isopropyl, butyl und 2-ethylhexyl.

In case of using a heterogeneous catalysis, the catalyst is fixed on a carrier or added to the reaction vessel.

In an alternative further development of the inventive process, the homogeneous catalyst is used in quantities of up to 10 wt.-%, based on the total mass of the reaction mixture, preferably in quantities from 0.01 wt.-% to 10 wt.-%, and particularly preferred from 0.02 wt.-% to 2 wt.-%.

It is further preferred if the heterogeneous catalyst is selected from dialkyl-tinoxides (R)₂SnO, whereby R is defined as above, preferably di-n-butyltinoxid.

It is more preferred to select the catalyst from Zeolite, silica gel, acidic silica bearing sulphonic acid (SiliaBond Propylsulfonic Acid [SCX-2] and SiliaBond Tosic Acid [SCX] [R60530B]), layered silicate like montmorillonite (in particular montmorillonite K10) and organic ion exchanger.

li is particularly preferred if the catalyst is selected from Zeolite HY 60 (SiO₂: Al₂O₃ 60 : 1, Zeolite HY 5.2 (SiO₂ : Al₂O₃ 5.2 : 1), β-zeolite (Si : Al of 30 : 1).

In a preferred embodiment of the invention, the heterogenous catalyst is used in quantities of 0.01 wt.-% to 15.0 wt.-% related to the total weight-mass of the reaction mixture, preferably 0.1 wt.-% to 12.0 wt.-%, more preferably 2.0 wt.-% to 10.0 wt.-%, particularly preferred 3.0 wt.-% to 8.0 wt.-%.

A further development provides the microwave-transparent zone being filled with an open-cell foam bearing or consisting of a heterogenous catalytically active species. By this feature combination, the catalytically active particles can be fixed on the foam, resulting in short contact times and a high catalytic selectivity of the reaction.

In a preferred embodiment of the invention, step b) is performed in a monomode microwave oven, in particular by irradiating the reaction mixture with microwaves in a microwave-transparent reaction tube which is conducted through an E01n cavity resonator with axial introduction of the microwaves, the length of the cavity resonator being such as to form a standing wave where n = 2 or more field maxima of the microwave.

The use of straight tubes as the heating tube (i.e. microwave-transparent reaction tube) has been found to be particularly useful. In a more preferred embodiment, the reaction mixture is heated in a microwave-transparent, straight tube, the longitudinal axis of which is in the direction of propagation of the microwaves of a monomode microwave applicator. By this embodiment, an accurate reaction control and energy input is achieved through defined consecutive heating zones at the maxima of the standing wave.

In another embodiment of the invention, the frequencies of the microwave radiation used are 915 MHz and 2.45 GHz and the microwave power to be injected into the cavity resonator is between 0.1 kW and 100 kW. The throughput and available capacities can be increased by changing from 2.45 GHz to 914 MHz due to a larger reaction zone for 915 MHz waves.

In this regard, it is preferred if the temperature in the microwave-transparent reaction zone is equal or between 120 °C and 200 °C, much preferable 150 °C to 190 °C and particularly preferred 160 °C to 180 °C.

In a further preferred embodiment, the residence time of the reaction mixture in the microwave-transparent reaction zone is 5 s to 150 s, much preferable 10 s to 100 s and particularly preferred 20 s to 60 s. This leads to a short exposure of the sensitive compounds like squalene to high temperatures, minimizing decomposition, isomerization or side reactions.

Squalene, which has been described above, is highly sensitive towards various forms of reactive oxygen species leading to different oxidized by-products.

The product comprising squalene according to Formula I as defined and described above can be used in a cosmetic product or a pharmaceutical product.

Therein, squalene according to Formula I is contained in concentrations of 1,0 wt.-% to 99,9 wt.-%, related to the total weight of the cosmetic or pharmaceutical product. These cosmetic or pharmaceutical products are free of impurities which may cause skin irritation or sensitisation.

This product is used as spreading agent in cosmetic products and produces a pleasant, silky feeling on the skin and helps to generate the skin's lipid barrier. In hair care products squalene is used has conditioner agent and in decorative cosmetics as dispersing agent of pigments in emulsion.

Furthermore, squalene is used in the pharmaceutical industry in ointment bases as a spreading agent and is used to enhance the activities in vaccines.

### Example 1: Concentration of squalene through esterification of soja-DDO with aqueous methanol

3,900 g soja-DDO (13.3 mol; molecular weight: 292,2 g/mol) and 2,110 g methanol, containing 5 % water, are mixed in a heated glass vessel. 4 g hydrochloric acid (30 %) is added to the emulsion.

The reaction mix is pumped into the continuous flow mono mode microwave reactor via a gear pump with a flow rate of 6 h/L. The mixture is heated by microwave radiation (energy input 1 kW) to 100 °C at a pressure of 2.5 bar.

The reaction mixture is removed from the reactor through a pressure release valve and cooled down to 60 °C by means of an ice bath. The lower phase (water, catalyst) is separated via a phase separator. The upper phase is transferred into a 3 L three necked flask and methanol is distilled off at 65 °C.

After removal of methanol, 1,000 ml water are added and the mixture is steered at 40 °V to 50 °C for 10 minutes.

In a subsequent second phase separation, the lower phase consisting of water and methanol is removed at 50 °C. 3,750 g methylester fraction, containing unsaponifiables (tocopherol, Squalene, sterols) is isolated with a yield of 90 % based on the initial soja-DDO.

1,000 g of the methylester fraction are added to a falling film evaporator at 200 °C and methylester is distilled of at 2 mbar. 845 g fatty acid methylester and 120 g of a dark red viscous liquid, which contains mainly tocopherols, squalene, and sterols as a concentrate, are received.

The separation of squalene was performed using Flash chromatography. The columns were packed with reverse-phase C-18 silica, and the mobile phase used was a mix of MeOH containing 2 wt.-% water and 2 wt.-% 2-propanol. The separation was controlled by measuring UV-Vis absorbance at 254 nm and 214 nm. Preliminary experiments to find the best gradient were conducted on a 100 g RediSep column. Prior to chromatography, samples were dissolved in CH₂Cl₂ and adsorbed on normal phase silica to be placed in a pre-column cartridge.

By Flash chromatography of 20 g of the concentrate from the esterification step (containing tocopherol, squalene and sterol) 11.1 g of squalene-rich fraction was isolated in 55.4 % yield. The squalene content according to figure I was 87,4 %. Only traces of isomerization compounds could be detected.

### Example 2: Concentration of squalene through esterification of soja-DDO with aqueous ethanol

3,900 g soja-DDO (13,3 mol; molecular weight: 292,2 g/mol) and 3,190 g ethanol, containing 10 % water, are mixed in a heated glass vessel. 4 g hydrochloric acid (30 %) is added to the emulsion.

The reaction mix is pumped into the continuous flow mono mode microwave reactor via a gear pump with a flow rate of 6 h/L. The mixture is heated by microwave radiation (energy input 1 kW) to 100 °C at a pressure of 2.5 bar.

The reaction mixture is removed from the reactor through a pressure release valve and cooled down to 60 °C by means of an ice bath. The lower phase (water, catalyst) is separated via a phase separator. The upper phase is transferred into a 3 L three necked flask and methanol is distilled off at 65 °C.

After removal of ethanol, 1,000 ml water are added and the mixture is steered at 40 °C to 50 °C for 10 minutes.

In a subsequent second phase separation the lower phase consisting of water and ethanol is removed at 50 °C. 4,150 g ethylester fraction, containing unsaponifiables (tocopherol, squalene, sterols) is isolated with a yield of 95 %.

1,000 g of the ethylester fraction are added to a falling film evaporator at 200 °C and ethylester is distilled of at 2 mbar. 885 g fatty acid ethylester and 148 g of a dark red viscous liquid, which contains mainly tocopherols, squalene, and sterols as a concentrate, are received.

The separation squalene from the concentrate performed using Flash chromatography according to example 1.

By Flash chromatography of 20 g of the concentrate (containing tocopherol, squalene and sterol) 12.1 g of squalene-rich fraction were isolated in 59,7 % yield. The squalene content according to formula I was 88,3 %. Only traces of isomerization compounds could be detected.

### Comparative example

### 3: Hydrogenation of squalene from the Flash-chromatography step to squalane

The hydrogenation of squalene was carried out in a microwave reactor under H₂ pressure using Pd/C 10 wt.-% as the catalyst. 4.1 g (10 mmol) squalene, dissolved in 50 ml ethanol, and 50 mg catalyst were placed in a 200 ml glass vial under magnetic stirring.

The reaction chamber was a 1 L PTFE cavity filled with 100 ml of water (20 wt.-% NaCl), which absorbs the radiation excess. Hydrogen was pourched through the reactor with a pressure of 5 bar and 100 °C for 1 hour.

After the reaction, the sample was filtered on paper and HPLC filters and analysed using GC-MS and ¹H-NMR to measure conversion and selectivity.

The conversion to squalene was quantitative with a selectivity of 98.4 %. Isomerization of cleaving products were not detected.

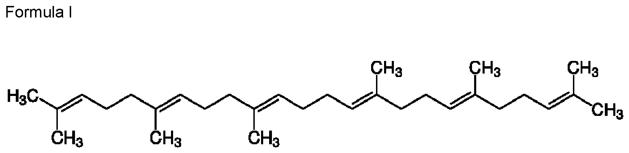

## Claims

1. A continuous flow, microwave assisted process for producing a product comprising squalene according to Formula I in an amount of equal or more than 50 wt.-% related to the total weight of the product, 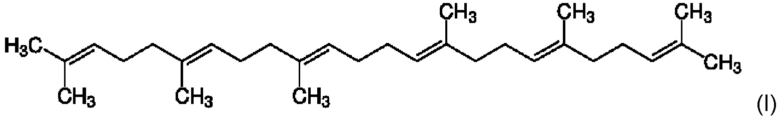 the process comprising the steps
a) preparing a reaction mixture containing an oily or fatty material of plant origin and aqueous methanol or aqueous ethanol,
b) continuously conveying of the reaction mixture through a microwave-transparent reaction zone and irradiating the reaction mixture with microwaves inside the reaction zone in the presence of an acidic catalyst, whereby the retention time of the reaction mixture in the microwave-transparent reaction zone is 1 s to 180 s, the temperature in the reaction zone being equal to or between 100 °C and 220 °C, the pressure in the reaction zone being 1.5 bar to 30 bar,
wherein a reaction of the reaction mixture takes place in the reaction zone resulting in a mixture of polar substances comprising glycerol, methanol or ethanol, and water, and non-polar substances comprising fatty acid methylesters or fatty acid ethylesters and unsaponifiable components,
c) releasing the pressure to ambient pressure,
d) after phase separation the polar phase is separated from the non-polar phase containing the non-polar substances,
e) separating fatty acid methylesters or fatty acid ethylesters from the non-polar phase by falling film evaporation or thin-layer evaporation,
f) separating squalene according to Formula I from the unsaponifiable components by molecular distillation, short-pass distillation, extraction or freeze separation.

2. The process according to claim 1, wherein step b) is performed in a monomode microwave oven, in particular by irradiating the reaction mixture with microwaves in a microwave-transparent reaction tube which is conducted through an E01n cavity resonator with axial introduction of the microwaves, the length of the cavity resonator being such as to form a standing wave where n = 2 or more field maxima of the microwave.

3. The process according to claims 1 or 2, wherein the frequencies of the microwave radiation used are 915 MHz and 2.45 GHz and the microwave power to be injected into the cavity resonator is between 0.1 kW and 100 kW.

4. The process according to any of claims 1 to 3, wherein the microwave-transparent zone is being filled with an open-cell foam bearing or consisting of a heterogenous catalytically active species.

## Patentansprüche

1. Kontinuierliches, mikrowellenunterstütztes Verfahren zur Herstellung eines Produkts, das Squalen gemäß Formel I in einer Menge von mindestens 50 Gew.-%, bezogen auf das Gesamtgewicht des Produkts, enthält, 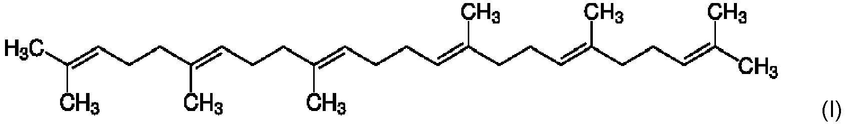 wobei das Verfahren die folgenden Schritte umfasst
a) Herstellen eines Reaktionsgemisches, das ein öliges oder fettiges Material pflanzlichen Ursprungs und wässriges Methanol oder wässriges Ethanol enthält,
b) kontinuierliches Fördern des Reaktionsgemisches durch eine mikrowellendurchlässige Reaktionszone und Bestrahlen des Reaktionsgemisches mit Mikrowellen innerhalb der Reaktionszone in Gegenwart eines sauren Katalysators, wobei die Verweilzeit des Reaktionsgemisches in der mikrowellendurchlässigen Reaktionszone 1 s bis 180 s beträgt, die Temperatur in der Reaktionszone gleich oder zwischen 100 °C und 220 °C liegt, der Druck in der Reaktionszone 1,5 bar bis 30 bar beträgt,
wobei in der Reaktionszone eine Reaktion des Reaktionsgemisches stattfindet, die zu einem Gemisch aus polaren Substanzen, die Glycerin, Methanol oder Ethanol und Wasser umfassen, und unpolaren Substanzen, die Fettsäuremethylester oder Fettsäureethylester und unverseifbare Bestandteile umfassen, führt,
c) Entlasten des Drucks auf Umgebungsdruck,
d) nach der Phasentrennung Abtrennen der polaren Phase von der die unpolaren Stoffe enthaltenden unpolaren Phase,
e) Abtrennen von Fettsäuremethylester oder Fettsäureethylester aus der unpolaren Phase durch Fallfilmverdampfung oder Dünnschichtverdampfung,
f) Abtrennen von Squalen gemäß Formel I aus den unverseifbaren Bestandteilen durch Molekularzerstäubung, Kurzzeitdestillation, Extraktion oder Gefriertrennung.

2. Verfahren nach Anspruch 1, wobei Schritt b) in einem monomodalen Mikrowellenofen durchgeführt wird, insbesondere durch Bestrahlen des Reaktionsgemisches mit Mikrowellen in einem mikrowellendurchlässigen Reaktionsrohr, das durch einen E01n-Hohlraumresonator mit axialer Einleitung der Mikrowellen geführt wird, wobei die Länge des Hohlraumresonators so bemessen ist, dass eine stehende Welle mit n = 2 oder mehr Feldmaxima der Mikrowelle entsteht.

3. Verfahren nach Anspruch 1 oder 2, wobei die Frequenzen der verwendeten Mikrowellenstrahlung 915 MHz und 2,45 GHz betragen und die in den Hohlraumresonator einzuspeisende Mikrowellenleistung zwischen 0,1 kW und 100 kW liegt.

4. Verfahren nach einem der Ansprüche 1 bis 3, wobei die mikrowellendurchlässige Zone mit einem offenzelligen Schaum gefüllt ist, der eine heterogene katalytisch aktive Spezies trägt oder aus einer solchen besteht.

## Revendications

1. Procédé à flux continu assisté par micro-ondes destiné à produire un produit comprenant du squalène selon la formule I en une quantité supérieure ou égale à 50 % en poids par rapport au poids total du produit, 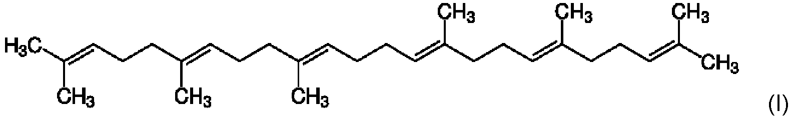 le procédé comprenant les étapes
a) de préparation d'un mélange réactionnel contenant un matériau huileux ou gras d'origine végétale et du méthanol aqueux ou de l'éthanol aqueux,
b) de transport continu du mélange réactionnel à travers une zone de réaction transparente aux micro-ondes et d'irradiation du mélange réactionnel avec des micro-ondes à l'intérieur de la zone de réaction en présence d'un catalyseur acide, où le temps de rétention du mélange réactionnel dans la zone de réaction transparente aux micro-ondes va de 1 s à 180 s, la température dans la zone de réaction est égale à ou est comprise entre 100 °C et 220 °C, la pression dans la zone de réaction va de 1,5 bar à 30 bar,
dans lequel une réaction du mélange réactionnel a lieu dans la zone de réaction conduisant à un mélange de substances polaires comprenant du glycérol, du méthanol ou de l'éthanol, et de l'eau, et de substances non polaires comprenant des esters méthyliques d'acide gras ou des esters éthyliques d'acide gras et des composants insaponifiables,
c) de libération de la pression à la pression ambiante,
d) après la séparation de phase, de séparation de la phase polaire de la phase non polaire contenant les substances non polaires,
e) de séparation d'esters méthyliques d'acide gras ou d'esters éthyliques d'acide gras de la phase non polaire par évaporation à ruissellement ou évaporation à couches minces,
f) de séparation du squalène de formule I des composants insaponifiables par distillation moléculaire, distillation à court passage, extraction ou séparation par congélation.

2. Procédé selon la revendication 1, dans lequel l'étape b) est réalisée dans un four à micro-ondes à mode unique, en particulier en irradiant le mélange réactionnel de micro-ondes dans un tube de réaction transparent aux micro-ondes qui est guidé à travers un résonateur à cavité E01n avec une introduction axiale des micro-ondes, la longueur du résonateur à cavité étant telle qu'elle forme une onde stationnaire où n = 2 ou plusieurs maxima de champ de la micro-onde.

3. Procédé selon les revendications 1 ou 2, dans lequel les fréquences du rayonnement micro-ondes utilisées sont de 915 MHz et de 2,45 GHz et la puissance des micro-ondes à injecter dans le résonateur à cavité est comprise entre 0,1 kW et 100 kW.

4. Procédé selon l'une quelconque de revendications 1 à 3, dans lequel la zone transparente aux micro-ondes est remplie d'une mousse à cellules ouvertes présentant ou comprenant une variété catalytiquement active hétérogène.
